# EUROPÄISCHE PATENTANMELDUNG

(11) **EP 1 459 710 A2**
(43) Veröffentlichungstag der Anmeldung: **22.09.2004**
(21) Anmeldenummer: 04001532.3
(22) Anmeldetag: 24.01.2004
(51) Int. Cl.: A61F 2/44, A61F 2/30

(54) **Implantat zum Einsetzen zwischen Wirbelkörper der Wirbelsäule**

(30) Priorität: 15.03.2003 DE 10311477
(71) Anmelder: Ulrich GmbH & Co. KG, 89081 Ulm (DE)
(72) Erfinder: Neumann, Carsten, Dr.med., 93007 Bad Abbach (DE)
(74) Vertreter: Hentrich, Swen, Dr.,Dipl.-Phys.

(57) **Zusammenfassung**

Die Erfindung betrifft ein Implantat zum Einsetzen zwischen Wirbelkörper der Wirbelsäule als Platzhalter für aus der Wirbelsäule entfernte Bandscheiben, Wirbel oder Wirbelteile. Das Implantat (1) besitzt ein erstes Implantatteil (2) und ein zweites Implantatteil (3), die in Richtung ihrer koaxialen Längsachsen zur Längenänderung des Implantats (1) gegeneinander verstellbar sind, sowie eine Ansatzplatte (5), die an dem freien Ende mindestens eines der beiden Implantatteile (2,3) in im wesentlichen zur Längsachse senkrechter Ausrichtung durch Mittel zur lösbaren Befestigung anschließbar ist.

## Beschreibung

Die Erfindung betrifft ein Implantat zum Einsetzen zwischen Wirbelkörper der Wirbelsäule als Platzhalter für aus der Wirbelsäule entfernte Bandscheiben, Wirbel oder Wirbelteile, mit einem ersten Implantatteil und einem zweiten Implantatteil, die in Richtung ihrer koaxialen Längsachsen zur Längenänderung des Implantats gegeneinander verstellbar sind.

Ein derartiges Implantat ist beispielsweise aus der DE 44 23 2057 A1 bekannt, das sich in der Praxis gut bewährt hat und durch die ihm eigene einfache Distraktionsmöglichkeit auszeichnet, die erreicht wird, indem die beiden auf dem mittleren Implantatteil angeordneten endständigen Implantatteile über ihre Gewindeverbindung gegenüber dem mittleren Implantatteil verschraubt und dadurch in der Höhe gemäß einer Spindel verstellt werden. Allerdings ist zu beachten, daß für die Distraktion ein relativ großer Platzbedarf erforderlich ist, um ein Werkzeug während der Operation verschwenken und somit die Implantatteile gegeneinander verdrehen zu können.

Der Erfindung liegt daher die Aufgabe zugrunde, ein Implantat der eingangs genannten Art so auszubilden, daß während der Operation eine individuelle Anpassung des Implantats an die in situ vorliegenden Gegebenheiten möglich ist zur Vereinfachung der Lagerhaltung hinsichtlich der Bereitstellung unterschiedlich dimensionierter Implantate und einer Erleichterung der während der Operation durchzuführenden Distraktion.

Diese Aufgabe wird gelöst durch ein Implantat der eingangs genannten Art, das gekennzeichnet ist durch eine Ansatzplatte, die an dem freien Ende mindestens eines der beiden Implantatteile in im wesentlichen zur Längsachse senkrechte Ausrichtung durch Mittel zur lösbaren Befestigung anschließbar ist.

Mit diesem Implantat ist der Vorteil verbunden, daß in einfacher Weise die Längenerstreckung des Implantats durch die Ansatzplatte geändert werden kann, so daß das Implantat selber in einer weniger feinen Abstufung bereitgestellt werden muß und darüber hinaus eine Vergrößerung des Bereiches vorliegt, der hinsichtlich der gewünschten Längenänderung zur Distraktion abgedeckt werden kann; das Ausmaß der Distraktion mit der dazu erforderlichen gegenseitigen Verstellung der Implantatteile ist um die Dicke der Ansatzplatte verringert.

Aus diesem Grunde ist es vorteilhaft, wenn die Dicke der Ansatzplatte 2,0 % bis 30,0 % der Höhe eines der Implantatteile entspricht.

Eine ganz besonders bevorzugte Ausführungsform der Erfindung ist dadurch gekennzeichnet, daß die Ansatzplatte die Außenkontur des Implantates überragt. Damit ist der große Vorteil verbunden, daß der effektive Wirkungsquerschnitt des Implantates nicht mehr allein durch dessen Außenkontur bestimmt ist, sondern zur Variation der Flächenpressung durch geeignete Wahl der Größe der Ansatzplatte angepaßt werden kann.

Für die einfache Handhabung während der Operation ist dabei entscheidend, daß in einfacher Weise die Verbindung der Ansatzplatte mit dem Implantat hergestellt werden und auch wieder gelöst werden kann. Dazu ist es günstig, wenn die Mittel zur lösbaren Befestigung eine in der Ansatzplatte ausgebildete Öffnung umfassen, so daß in einfachster Weise eine Steckverbindung hergestellt werden kann durch Aufstecken der Ansatzplatte mit ihrer Öffnung auf das Implantat. Dabei wird vorzugsweise die Ansatzplatte außenseitig auf das Implantat aufgesteckt, so daß die Form der Öffnung an die Außenkontur des Implantates angepaßt ist.

Nach einer Alternative der Erfindung ist vorgesehen, daß die Öffnung im Massenschwerpunkt der Ansatzplatte ausgebildet ist, so daß sich eine symmetrische Konfiguration bezüglich der Längsachse des Implantates ergibt.

Es besteht aber gleichfalls auch die Möglichkeit, daß die Öffnung außerhalb des Massenschwerpunktes der Ansatzplatte ausgebildet ist, um so Gegebenheiten gerecht werden zu können, bei denen eine einseitig vergrößerte Abstützungsfläche des Wirbelkörpers durch das Implantat gewünscht ist, wie dies beispielsweise auch dann der Fall sein kann, wenn das Implantat zu zweien, paarweise eingesetzt wird, um den Wirbelzwischenraum zwischen zwei Wirbelkörpern zu überbrücken.

Eine verbesserte Kontaktfläche zwischen dem Implantat und dem Wirbelkörper wird dadurch bereitgestellt, daß die dem Wirbelkörper zugewandte Oberfläche der Ansatzplatte konvex gewölbt ist.

Im Rahmen der Erfindung ganz besonders bevorzugt ist weiterhin eine Ausführungsform, die dadurch gekennzeichnet ist, daß die Ansatzplatte mit den Mitteln zur Befestigung zweifach vorgesehen und jeweils einem der Implantatteile zugeordnet ist, da so die Vorteile der Erfindung bei jedem dem Implantat angrenzenden Wirbelkörper ausgenutzt werden können.

Um die Handhabung des Implantats mit der Ansatzplatte unter den Bedingungen einer Operation möglichst einfach zu gestalten, ist vorgesehen, daß die Mittel zur Befestigung durch eine zwischen der Ansatzplatte und dem Implantatteil ausgebildete Steckverbindung und einem Rastsitz gebildet sind. Dies ist beispielsweise dadurch möglich, daß die Mittel zur Befestigung durch einen Bajonettverschluß gebildet sind. Alternativ besteht die Möglichkeit, daß die Mittel zur Befestigung eine an dem freien Ende des Implantatteils ausgebildete Nut sowie eine in einer Nut der Öffnung der Ansatzplatte gelagerte Feder umfassen, die beim Aufstecken der Ansatzplatte auf das Implantatteil federnd ausweichen und als Rastglied in die Nut des Implantatteils eintreten kann.

Um eine sichere Verankerung des Implantats in den Wirbelkörpern zu erleichtern, ist es möglich, daß auf der dem Wirbelkörper zugewandten Seite der Ansatzplatte Dorne und/oder Schneiden angeordnet sind.

Eine besonders große Variabilität des als modularen Systems aufgebauten Implantats wird dadurch erreicht, daß die Ansatzplatte als Polygon oder alternativ gerundet oder alternativ sternförmig gestaltet ist. Insbesondere die sternförmige Gestaltung der Ansatzplatte zeichnet sich dabei durch die Eigenschaft aus, daß die Ansatzplatte keine geschlossene Fläche darstellt als scharfe Trennebene zwischen dem Wirbelkörper und dem unterhalb der Ansatzplatte angeordneten Implantat. Damit ist die Möglichkeit geschaffen, daß sich eine knöcherne Verbindung zwischen den beiden Wirbelkörpern ausbilden und das Implantat besonders gut einwachsen kann, insbesondere durch die unterstützende Verwendung von Knochenspänen, Knochenzement oder dergleichen.

Der vorstehend genannte Vorteil ist nicht nur gegeben, wenn die Ansatzplatte sternförmig gestaltet ist, sondern auch, wenn in der Ansatzplatte eine Durchtrittsöffnung ausgebildet ist. Um eine gute knöcherne Durchdringung des Implantates zu erleichtern, ist es zweckmäßig, daß die Durchtrittsöffnung mehrfach vorgesehen ist.

Zweckmäßig ist weiterhin, wenn die Durchtrittsöffnung sich bis zum äußeren Rand der Ansatzplatte erstreckt, da so die Zugänglichkeit zur Anlagerung von knochenbildendem Material vereinfacht ist und eine vergrößerte Kontaktfläche bzw. -länge zur Anlagerung dieses Materials vorliegt.

Nach einer weiteren Ausführungsform der Erfindung ist vorgesehen, daß von der Öffnung ausgehende Plattenstege die Durchtrittsöffnungen bilden und begrenzen. Die Stabilität einer derartig gestalteten Ansatzplatte wird verbessert, indem die Plattenstege an ihren freien Enden miteinander verbunden sind zur Bildung des Randes der Ansatzplatte.

Eine weitere ganz besonders bevorzugte Ausführungsform der Erfindung ist dadurch gekennzeichnet, daß dem ersten Implantatteil ein drehbarer Gewindering zugeordnet ist, der mit einem Ringgewinde in ein dem zweiten Implantatteil zugeordnetes Gewinde eingreift, und daß der Gewindering mit einem Kegelradverzahnung versehen ist, da bei einem derartig gestalteten Implantat der Platzbedarf zur Längenänderung des Implantates, also der Distraktion, durch Vermeiden einer Schwenkbewegung reduziert ist.

Zweckmäßigerweise ist zur Anpassung an die anatomischen Gegebenheiten die dem Wirbelkörper zugewandte Seite der Ansatzplatte und/oder die dem Implantatteil zugewandte Seite der Ansatzplatte geneigt zur Längsachse orientiert, wobei der Neigungswinkel zwischen 3° und 45° beträgt.

Die gute Anpassung an die Anatomie bedingt, daß die Drehlage der Ansatzplatte um die Längsachse gegenüber dem Implantatteil festlegbar ist, wozu vorgesehen ist, daß in Umfangsrichtung in Schrittweiten von 10° bis 45° Rastsitze zwischen der Ansatzplatte und dem Implantatteil ausgebildet sind.

Im folgenden wird die Erfindung an in der Zeichnung dargestellten Ausführungsbeispielen näher erläutert; es zeigen:
- Fig. 1: eine perspektivische Darstellung eines aus einem ersten Implantatteil und einem zweiten Implantatteil bestehenden Implantates mit zwei an den freien Enden der beiden Implantatteile befestigten Ansatzplatten,
- Fig. 2: das Implantat aus Figur 1 in einer Seitenansicht,
- Fig. 3: eine der Figur 1 entsprechende Darstellung eines Implantates mit nicht in ihren Massenschwerpunkten an den beiden Implantatteilen befestigten Ansatzplatten,
- Fig. 4: eine der Figur 2 entsprechende Darstellung des Implantates aus Figur 3,
- Fig. 5: eine Ansicht aus Richtung des Pfeiles V aus Figur 4,
- Fig. 6: eine Draufsicht auf das Implantat gemäß Figur 3,
- Fig. 7: eine der Figur 1 entsprechende Darstellung einer weiteren alternativen Ausführungsform,
- Fig. 8: eine Seitenansicht des Implantates aus Figur 7,
- Fig. 9: eine der Figur 1 entsprechende Darstellung einer weiteren alternativen Ausführungsform,
- Fig. 10: eine isolierte, perspektivische Darstellung einer Ansatzplatte,
- Fig. 11: eine Draufsicht auf eine weitere Ansatzplatte,
- Fig. 12: eine isolierte, perspektivische Darstellung einer weiteren Ansatzplatte,
- Fig. 13: eine isolierte, perspektivische Darstellung einer weiteren Ansatzplatte,
- Fig. 14: eine Draufsicht auf eine weitere Ansatzplatte,
- Fig. 15: eine Draufsicht auf eine weitere Ansatzplatte,
- Fig. 16: eine isolierte, perspektivische Darstellung einer weiteren, Plattenstege aufweisenden Ansatzplatte,
- Fig. 17: eine Draufsicht auf eine weitere, Plattenstege aufweisende Ansatzplatte,
- Fig. 18: eine der Figur 17 entsprechende Darstellung einer weiteren Ausführungsform einer Ansatzplatte,
- Fig. 19: eine der Figur 17 entsprechende Darstellung einer weiteren Ausführungsform einer Ansatzplatte,
- Fig. 20: eine isolierte, perspektivische Darstellung einer weiteren Ansatzplatte,
- Fig. 21: eine der Figur 20 entsprechende Darstellung einer weiteren Ausführungsform der Ansatzplatte,
- Fig. 22: eine der Figur 20 entsprechende Darstellung einer weiteren Ausführungsform einer Ansatzplatte,
- Fig. 23: eine isolierte, perspektivische Darstellung einer an die Außenkontur des Implantates angepaßte Ansatzplatte,
- Fig. 24: eine Seitenansicht der Ansatzplatte aus Figur 23,
- Fig. 25: eine Draufsicht auf die Ansatzplatte gemäß Figur 23,
- Fig. 26: ein der Fig. 22 entsprechende Darstellung einer Ansatzplatte mit nicht-planparallelen Oberflächen,
- Fig. 27: eine Seitenansicht der Ansatzplatte aus Fig. 26,
- Fig. 28: eine Draufsicht auf eine Ansatzplatte,
- Fig. 29: den Schnitt XXIX-XXIX aus Fig. 28,
- Fig. 30: eine Seitenansicht der Ansatzplatte aus Fig. 28, und
- Fig. 31: eine der Fig. 1 entsprechende Darstellung eines Implantats mit zwei Ansatzplatten gemäß Fig. 28.

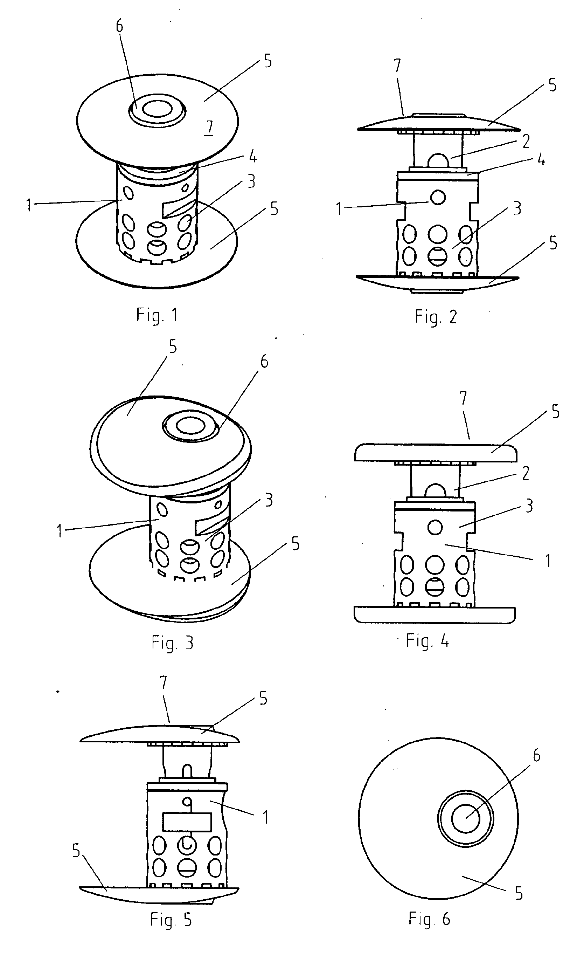

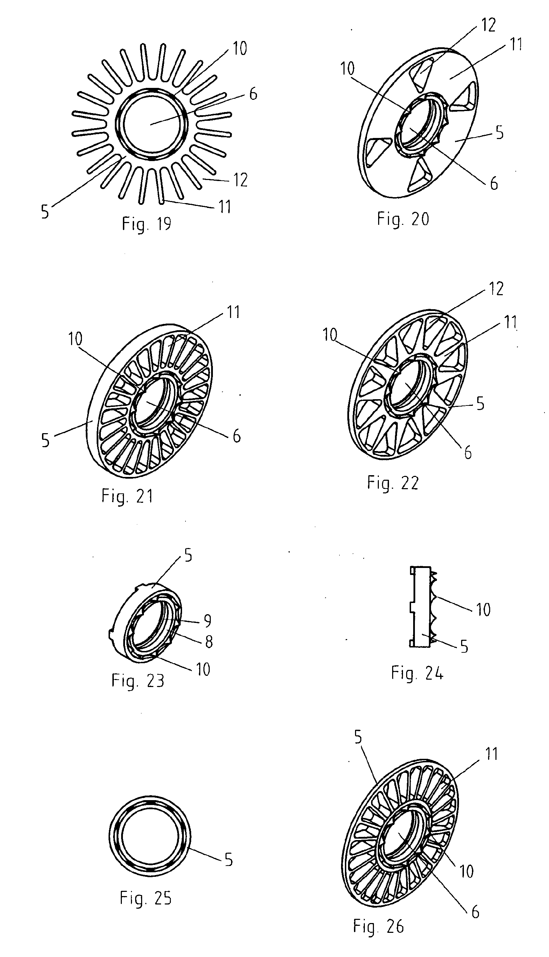

In den Figuren 1 und 2 ist ein Implantat 1 dargestellt, das zum Einsetzen zwischen in der Zeichnung selber nicht dargestellte Wirbelkörper der Wirbelsäule als Platzhalter für aus der Wirbelsäule entfernte Bandscheiben, Wirbel oder Wirbelteile dient. Das Implantat 1 umfaßt ein erstes Implantatteil 2 und ein zweites Implantatteil 3, die in Richtung ihrer koaxialen Längsachsen zur Längenänderung des Implantates 1 gegeneinander verstellbar sind. Bei dem in der Zeichnung dargestellten Ausführungsbeispiel ist dem zweiten Implantatteil 3 ein drehbarer Gewindering 4 zugeordnet, der mit einem Ringgewinde in ein dem ersten Implantatteil 2 zugeordnetes Gewinde eingreift, wobei der Gewindering 4 mit einer Kegelradverzahnung versehen ist, so daß durch ein an die Kegelradverzahnung angesetztes zweites Kegelrad die Längenänderung des Implantates 1 bewirkt werden kann. Das Implantat 1 weist weiterhin mindestens eine, in dem gezeigten Ausführungsbeispiel insgesamt zwei Ansatzplatten 5 auf, die an den freien Enden der beiden Implantatteile 2,3 in im wesentlichen zur Längsachse des Implantates 1 senkrechter Ausrichtung durch Mittel zur lösbaren Befestigung auswechselbar angeschlossen ist. Die Ansatzplatte 5 ist also mit den Mitteln zur Befestigung zweifach vorgesehen und jeweils einem der Implantatteile 2,3 zugeordnet, wobei die Dicke der Ansatzplatte 5 2,0 % bis 30,0 % der Höhe eines der Implantatteile 2,3 entspricht, so daß insbesondere durch die in den Figuren 23 bis 24 isoliert dargestellte Ansatzplatte 5 im wesentlichen eine Änderung der Länge des Implantates 1 bewirkt werden kann, während die übrigen in der Zeichnung dargestellten Ansatzplatten 5 sich dadurch auszeichnen, daß diese die Außenkontur des Implantates 1 überragen und so für das Implantat 1 eine vergrößerte Anlagefläche gegenüber dem angrenzenden Wirbelkörper bereitstellen.

Die Mittel zur lösbaren Befestigung umfassen eine in der Ansatzplatte ausgebildete Öffnung 6, die an die Außenkontur des Implantates 1 angepaßt ist, so daß die Ansatzplatte 5 in einfachster Weise auf das Implantat 1 aufgesteckt werden kann. Bei dem beispielsweise in den Figuren 1 und 2 dargestellten Ausführungsbeispiel ist die Öffnung 6 im Massenschwerpunkt der Ansatzplatte 5 ausgebildet, während die Figuren 3 bis 6 eine Ausführungsform zeigen, bei der die Öffnung 6 außerhalb des Massenschwerpunktes der Ansatzplatte 5 ausgebildet ist.

Die Figuren 2 und 5 lassen dabei erkennen, daß die dem Wirbelkörper zugewandte Oberfläche 7 der Ansatzplatte 5 konvex gewölbt ist.

Die Figuren 23 bis 25 zeigen, daß die Steckverbindung durch einen Rastsitz ergänzt ist, und zwar konkret gebildet durch eine an dem freien Ende des Implantatteils 2,3 ausgebildete Nut sowie eine in einer Nut 8 der Öffnung 6 der Ansatzplatte 5 gelagerte Feder 9. Insbesondere Figur 24 läßt erkennen, daß auf der dem Wirbelkörper zugewandten Seite der Ansatzplatte 5 Dorne 10 angeordnet sind, die auch als Schneiden mit in Umfangsrichtung längerer Ausdehnung gestaltet sein können.

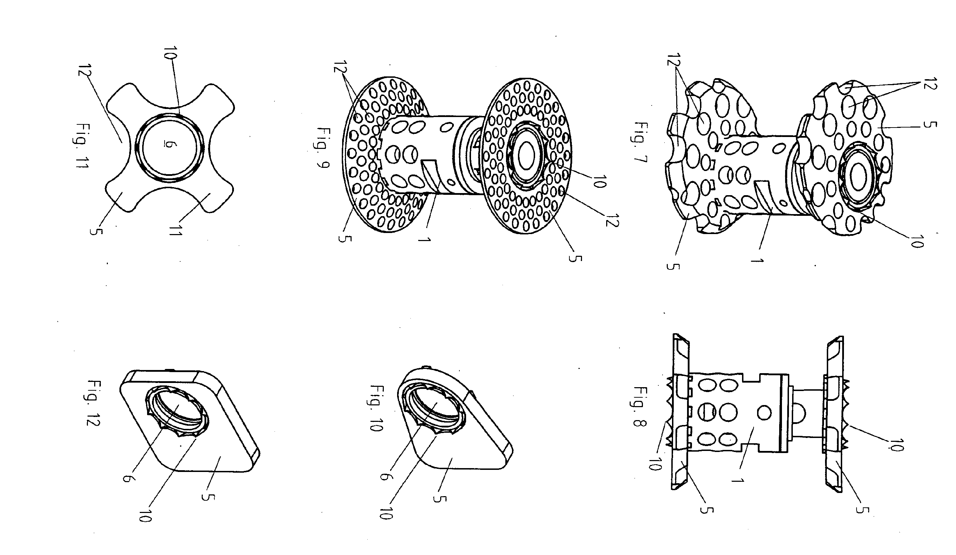

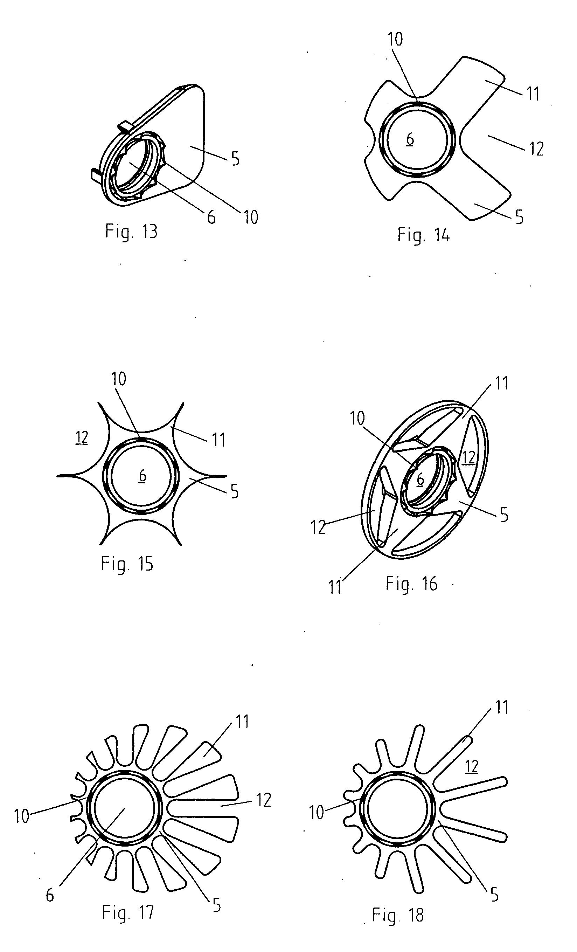

Hinsichtlich der Gestaltung der Ansatzplatte 5 gibt es vielfältige Variationsmöglichkeiten, um so den konkret vorliegenden Gegebenheiten bei einer Operation Rechnung tragen zu können; Figur 10 zeigt ebenso wie Figur 13 eine im wesentlichen dreieckige Gestaltung der Ansatzplatte 5 mit abgerundeten Ecken, während Figur 12 eine rechteckige Gestaltung zeigt, allgemein aber jedes Polygon realisiert werden kann, sofern dies anatomisch bzw. operativ gewünscht ist. Figur 11 ebenso wie Figur 14 und 15 zeigen eine sternförmige Gestaltung der Ansatzplatte 5, bei der die Bereiche zwischen Plattenstegen 11 als Durchtrittsöffnungen 12 aufgefaßt werden können, die bei der gegebenen radialen Erstreckung der Plattenstege 11 dennoch eine starke Annäherung an die Außenkontur des Implantates 1 mit beispielsweise Knochenzement ermöglichen. Insgesamt dient die vielfältige Gestaltung der Ansatzplatte 5 auch einer verbesserten Abstützung des Wirbelkörpers durch Ermöglichung eines großflächigen Kontaktes. Dadurch erstreckt sich in Figur 11 die Mehrzahl der in Figur 7 eingebettet dargestellten Durchtrittsöffnungen 12 bis zu dem Rand der Ansatzplatte 5. Die in den Figuren 17 bis 22 dargestellten Ausführungsformen lassen sich in einfachster Weise beschreiben durch die von der Öffnung 6 der Ansatzplatte 5 ausgehenden Plattenstege 11, die die Durchtrittsöffnungen bilden und begrenzen, wobei in den Figuren 21 und 22 die freien Enden der Plattenstege 11 miteinander verbunden sind zur Bildung des Randes der Ansatzplatte 5.

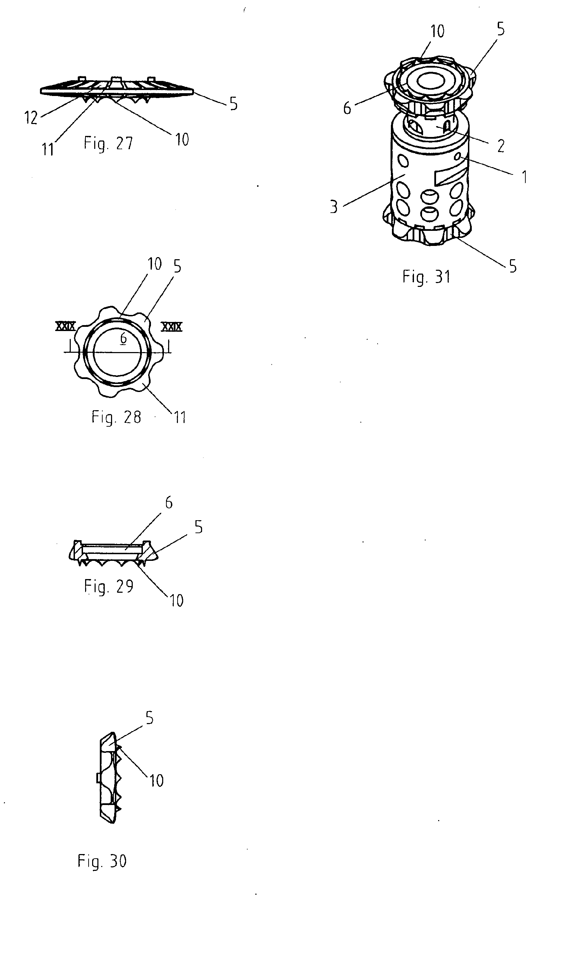

Die Fig. 26 und 27 lassen erkennen, daß die dem Wirbelkörper zugewandte Seite der Ansatzplatte 5 und die dem Implantatteil 2,3 zugewandte Seite der Ansatzplatte 5 geneigt zur Längsachse orientiert sind, so daß das Implantat 1 besser an die während der Operation vorgefundenen anatomischen Gegebenheiten angepaßt werden kann, wobei zu diesem Ziel Ansatzplatten 5 bereitgehalten werden, deren Neigungswinkel zwischen 3° und 45° beträgt.

Um die gute Anpassung dauerhaft zu sichern, ist die Drehlage der Ansatzplatte 5 um die Längsachse gegenüber dem Implantatteil 2,3 festlegbar, wozu in Umfangsrichtung in Schrittweiten von 10° bis 45° Rastsitze zwischen der Ansatzplatte 5 und dem Implantatteil 2,3 ausgebildet sind.

## Patentansprüche

1. Implantat zum Einsetzen zwischen Wirbelkörper der Wirbelsäule als Platzhalter für aus der Wirbelsäule entfernte Bandscheiben, Wirbel oder Wirbelteile, mit einem ersten Implantatteil (2) und einem zweiten Implantatteil (3), die in Richtung ihrer koaxialen Längsachsen zur Längenänderung des Implantats (1) gegeneinander verstellbar sind, **gekennzeichnet durch** eine Ansatzplatte (5), die an dem freien Ende mindestens eines der beiden Implantatteile (2,3) in im wesentlichen zur Längsachse senkrechter Ausrichtung **durch** Mittel zur lösbaren Befestigung anschließbar ist.

2. Implantat nach Anspruch 1, **dadurch gekennzeichnet, daß** die Dicke der Ansatzplatte (5) 2,0 % bis 30,0 % der Höhe eines der Implantatteile (2,3) entspricht.

3. Implantat nach Anspruch 1 oder 2, **dadurch gekennzeichnet, daß** die Ansatzplatte (5) die Außenkontur des Implantats (1) überragt.

4. Implantat nach Anspruch 3, **dadurch gekennzeichnet, daß** die Mittel zur lösbaren Befestigung eine in der Ansatzplatte (5) ausgebildete Öffnung (6) umfassen.

5. Implantat nach Anspruch 4, **dadurch gekennzeichnet, daß** die Form der Öffnung (6) an die Außenkontur des Implantats (1) angepaßt.

6. Implantat nach Anspruch 4 oder 5, **dadurch gekennzeichnet, daß** die Öffnung (6) im Massenschwerpunkt der Ansatzplatte (5) ausgebildet ist.

7. Implantat nach Anspruch 4 oder 5, **dadurch gekennzeichnet, daß** die Öffnung (6) außerhalb des Massenschwerpunkts der Ansatzplatte (5) ausgebildet ist.

8. Implantat nach einem der Ansprüche 1 bis 7, **dadurch gekennzeichnet, daß** die dem Wirbelkörper zugewandte Oberfläche (7) der Ansatzplatte (5) konvex gewölbt ist.

9. Implantat nach einem der Ansprüche 1 bis 8, **dadurch gekennzeichnet, daß** die Ansatzplatte (5) mit den Mitteln zur Befestigung zweifach vorgesehen und jeweils einem der Implantatteile (2,3) zugeordnet ist.

10. Implantat nach einem der Ansprüche 4 bis 9, **dadurch gekennzeichnet, daß** die Mittel zur Befestigung durch eine zwischen der Ansatzplatte (5) und dem Implantatteil (2,3) ausgebildete Steckverbindung und einem Rastsitz gebildet sind.

11. Implantat nach Anspruch 10, **dadurch gekennzeichnet, daß** die Mittel zur Befestigung durch einen Bajonettverschluß gebildet sind.

12. Implantat nach einem der Ansprüche 4 bis 9, **dadurch gekennzeichnet, daß** die Mittel zur Befestigung eine an dem freien Ende des Implantatteils (2,3) ausgebildete Nut sowie eine in einer Nut (8) der Öffnung (6) der Ansatzplatte (5) gelagerte Feder (9) umfassen.

13. Implantat nach einem der Ansprüche 1 bis 12, **dadurch gekennzeichnet, daß** auf der dem Wirbelkörper zugewandten Seite der Ansatzplatte (5) Dorne (10) und/oder Schneiden angeordnet sind.

14. Implantat nach einem der Ansprüche 3 bis 13, **dadurch gekennzeichnet, daß** die Ansatzplatte (5) als Polygon gestaltet ist.

15. Implantat nach einem der Ansprüche 3 bis 14, **dadurch gekennzeichnet, daß** die Ansatzplatte (5) gerundet gestaltet ist.

16. Implantat nach einem der Ansprüche 3 bis 15, **dadurch gekennzeichnet, daß** die Ansatzplatte (5) sternförmig gestaltet ist.

17. Implantat nach einem der Ansprüche 3 bis 16, **dadurch gekennzeichnet, daß** in der Ansatzplatte (5) eine Durchtrittsöffnung (12) ausgebildet ist.

18. Implantat nach Anspruch 17, **dadurch gekennzeichnet, daß** die Durchtrittsöffnung (12) mehrfach vorgesehen ist,

19. Implantat nach Anspruch 18, **dadurch gekennzeichnet, daß** die Durchtrittsöffnung (12) sich bis zum äußeren Rand der Ansatzplatte (5) erstreckt.

20. Implantat nach Anspruch 18 oder 19, **dadurch gekennzeichnet, daß** von der Öffnung ausgehende Plattenstege (11) die Durchtrittsöffnungen (12) bilden und begrenzen.

21. Implantat nach Anspruch 20, **dadurch gekennzeichnet, daß** die Plattenstege (11) an ihren freien Enden miteinander verbunden sind zur Bildung des Randes der Ansatzplatte (5).

22. Implantat nach einem der Ansprüche 1 bis 21, **dadurch gekennzeichnet, daß** dem zweiten Implantatteil (3) ein drehbarer Gewindering (4) zugeordnet ist, der mit einem Ringgewinde in ein dem ersten Implantatteil (2) zugeordnetes Gewinde eingreift, und daß der Gewindering (4) mit einer Kegelradverzahnung versehen ist.

23. Implantat nach einem der Ansprüche 1 bis 22, **dadurch gekennzeichnet, daß** die dem Wirbelkörper zugewandte Seite der Ansatzplatte (5) geneigt zur Längsachse orientiert ist.

24. Implantat nach einem der Ansprüche 1 bis 23, **dadurch gekennzeichnet, daß** die dem Implantatteil (2,3) zugewandte Seite der Ansatzplatte (5) geneigt zur Längsachse orientiert ist.

25. Implantat nach Anspruch oder 24, **dadurch gekennzeichnet, daß** der Neigungswinkel zwischen 3° und 45° beträgt.

26. Implantat nach einem der Ansprüche 23 bis 25, **dadurch gekennzeichnet, daß** die Drehlage der Ansatzplatte (5) um die Längsachse gegenüber dem Implantatteil (2,3) festlegbar ist.

27. Implantat nach Anspruch 26, **dadurch gekennzeichnet, daß** in Umfangsrichtung in Schrittweiten von 10° bis 45° Rastsitze zwischen der Ansatzplatte (5) und dem Implantatteil (2,3) ausgebildet sind.
